(19) Europäisches Patentamt / European Patent Office / Office européen des brevets

(11) **EP 4 450 810 B1**

(12) **EUROPEAN PATENT SPECIFICATION**

(45) Date of publication and mention
of the grant of the patent:
**03.06.2026 Bulletin 2026/23**

(21) Application number: **24171564.8**

(22) Date of filing: **22.04.2024**

(51) International Patent Classification (IPC):
*F04B 13/00* (2006.01)   *F04B 49/035* (2006.01)
*F04B 49/06* (2006.01)   *F04B 49/08* (2006.01)
*F04B 53/10* (2006.01)

(52) Cooperative Patent Classification (CPC):
**F04B 49/08; F04B 13/00; F04B 49/035;
F04B 49/06; F04B 53/10;** E02F 9/2296;
F04B 49/002; F15B 11/0423

(54) **ELECTRICALLY DIRECT CONTROLLED VARIABLE DISPLACEMENT PUMPS**

ELEKTRISCH DIREKT GESTEUERTE VERSTELLPUMPEN

POMPES VOLUMÉTRIQUES VARIABLES À COMMANDE ÉLECTRIQUE DIRECTE

(84) Designated Contracting States:
AL AT BE BG CH CY CZ DE DK EE ES FI FR GB GR HR HU IE IS IT LI LT LU LV MC ME MK MT NL NO PL PT RO RS SE SI SK SM TR

(30) Priority: **21.04.2023 US 202318137591**

(43) Date of publication of application:
**23.10.2024 Bulletin 2024/43**

(73) Proprietor: **Hamilton Sundstrand Corporation**
**Charlotte, NC 28217-4578 (US)**

(72) Inventors:
• SUSCA, Ryan Prescott
  Windsor, 06095 (US)
• SHOEMAKER, Mark W.
  Pecatonica, IL, 61063 (US)

(74) Representative: **Dehns**
**10 Old Bailey**
**London EC4M 7NG (GB)**

(56) References cited:
US-A- 5 806 300       US-A1- 2008 289 338
US-A1- 2014 072 457   US-A1- 2018 372 006
US-A1- 2022 372 968

## Description

### BACKGROUND

#### 1. Field

The present disclosure relates to pump control, and more particularly to control for variable displacement pumps (VDPs).

#### 2. Description of Related Art

In a pump, the turn-down ratio is the ratio of the pump's maximum flow to its minimum flow. In fuel delivery systems using a variable displacement pump (VDP), often the pump is subject to a high turn-down ratio. This can drive a pump design with a less than optimal pump efficiency throughout the operating range as a tradeoff for ensuring the turn-down ratio needed. For example, it is beneficial to pump design to minimize this turn-down ratio to be less than 4:1. But large displacement VDPs can struggle to function with such high turn down ratios in typical fuel metering systems because minimum pump pressure flows to support cooling the VDP are typically higher than the lowest flow for fuel burners, e.g. in gas turbine engines such as on aircraft. Control for VDPs is disclosed in US 2018/372006 A1 and US 2014/072457 A1. US2014/0072457 A1 discloses a fuel metering system in which a variable displacement pump is controlled by an electromechanical actuator, and a pump output is regulated using signals from position and pressure sensors under electronic control. This system does not include a flow-based recirculation arrangement, a bypass valve for controlled return flow, or a shut-off valve that initiates recirculation when delivered flow becomes too low.

The conventional techniques have been considered satisfactory for their intended purpose. However, there is an ever present need for improved systems and methods for control of VDPs. This disclosure provides a solution for this need.

### SUMMARY

A system according to the invention is defined by claim 1.

The controller can be configured to control the BPV to maintain a baseline flow through the BPV under a first condition wherein requested flow from the downstream system is above than the predetermined low threshold, and to control the BPV to increase the flow through the BPV above the baseline flow for a second flow condition wherein requested flow from the downstream system is at or below the predetermined low threshold.

A first electrohydraulic servo valve (EHSV) can be connected in fluid communication with the BPV by a first control line. The first EHSV can be connected in fluid communication with both the inlet line and with the outlet line through respective connection lines. The first EHSV can be operatively connected to the controller for active control of the first EHSV to actuate the BPV. A first MPSOV line can connect the MPSOV to the first control line for fluid communication. A second MPSOV line can connect the MPSOV to the inlet line for fluid communication. An MPSOV control line can connect the MPSOV in fluid communication with the inlet line through a fixed throttle. A solenoid valve (SOL) can be connected in fluid communication with the outlet line and with the MPSOV control line for actuating the MPSOV between first and second states. The MPSOV can include an MPSOV valve member with a first position in the MPSOV that connects the first and second MPSOV lines in fluid communication and blocks flow through the outlet line, and a second position that blocks fluid communication of the first and second MPSOV lines and allows flow though the outlet line.

A first position sensor can be operatively connected to the BPV to provide sensor output indicative of position of a valve member of the BPV. The first position sensor can be operatively connect the controller to provide feedback for controlling the BPV. A second EHSV ban be connected in fluid communication with the variable displacement mechanism by a second control line for control of flow through the VDP. The second EHSV can be connected in fluid communication with both the inlet line and with the outlet line through respective connection lines. The second EHSV can be operatively connected to the controller for active control of the second EHSV to actuate the variable displacement mechanism.

A second position sensor can be operatively connected to the variable displacement mechanism to provide sensor output indicative of position of the variable displacement mechanism. The second position sensor can be operatively connect the controller to provide feedback for controlling the variable displacement mechanism. A pressure sensor can be operatively connected to the outlet line to generate sensor output indicative of pressure in the outlet line. The pressure sensor can be operatively connected to the controller for active control of the variable displacement mechanism and/or of the BPV based on pressure in the outlet line.

The sensor output can be indicative of flow demanded by the downstream system supplied by the outlet line.

The FSV can include an FSV inlet, an FSV outlet, and a valve member. A biasing member can bias the valve member in a first direction. Pressure of flow through the FSV from the FSV inlet to the FSV outlet can bias the valve member in a second direction opposite the first direction.

The sensor can include a position sensor operatively connected to monitor position of the valve member in the FSV to generate the sensor data. The FSV can include a pressure port on a side of the valve member opposite from the FSV inlet and the FSV outlet. A pressure line can connect the FSV outlet in fluid communication with the pressure port.

A BPV control line can connect the BPV in fluid communication with the inlet line through a first fixed throttle. A first MPSOV line can connect the MPSOV in fluid communication with the BPV control line. A second MPSOV line can connect the MPSOV to the outlet line for fluid communication. An MPSOV control line can connect the MPSOV in fluid communication with the inlet line through a fixed throttle. A solenoid valve (SOL) can be connected in fluid communication with the outlet line and with the MPSOV control line for actuating the MPSOV between first and second states. The MPSOV can include an MPSOV valve member with a first position in the MPSOV that disconnects the first and second MPSOV lines from being in fluid communication and blocks flow through the outlet line, and a second position that connects the first and second MPSOV lines in fluid communication and allows flow though the outlet line.

A method according to the invention is defined by claim 9.

These and other features of the systems and methods of the subject disclosure will become more readily apparent to those skilled in the art from the following detailed description of the preferred embodiments taken in conjunction with the drawings.

## BRIEF DESCRIPTION OF THE DRAWINGS

So that those skilled in the art to which the subject disclosure appertains will readily understand how to make and use the devices and methods of the subject disclosure without undue experimentation, preferred embodiments thereof will be described in detail herein below with reference to certain figures, wherein:

Fig. 1 is a schematic view of an embodiment of a system constructed in accordance with the present disclosure, showing the connections of the variable displacement pump (VDP) and a bypass valve (BPV), with the minimum pressure and shutoff valve (MPSOV) for shutting of flow to the downstream systems such as a gas generator of a gas turbine engine, with active control of the BPV; and

Fig. 2 is a schematic view of another embodiment of the system of Fig. 1, showing the MPSOV connected with passive control of the BPV.

## DETAILED DESCRIPTION OF THE PREFERRED EMBODIMENTS

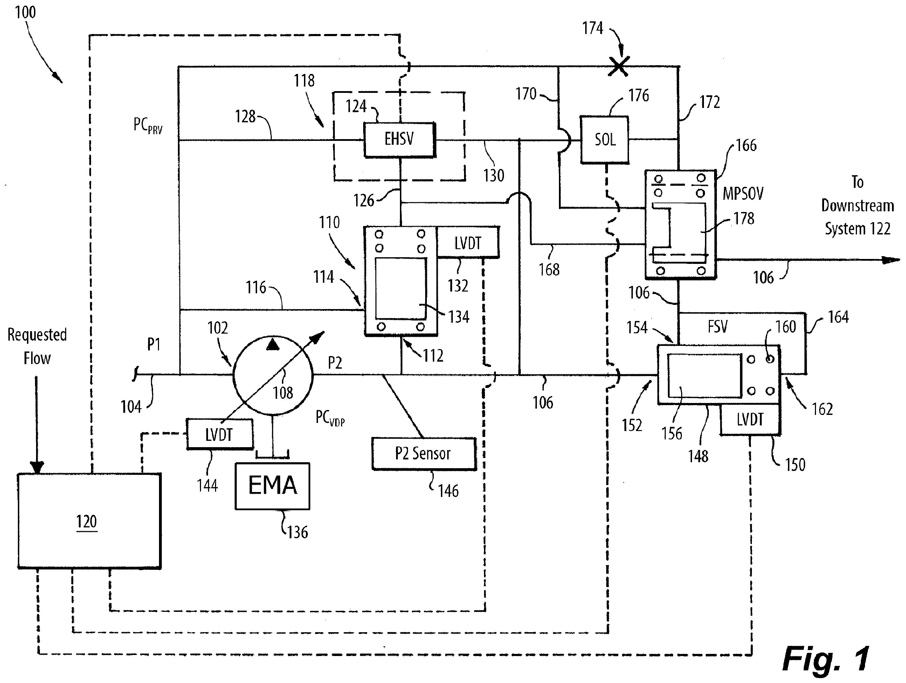

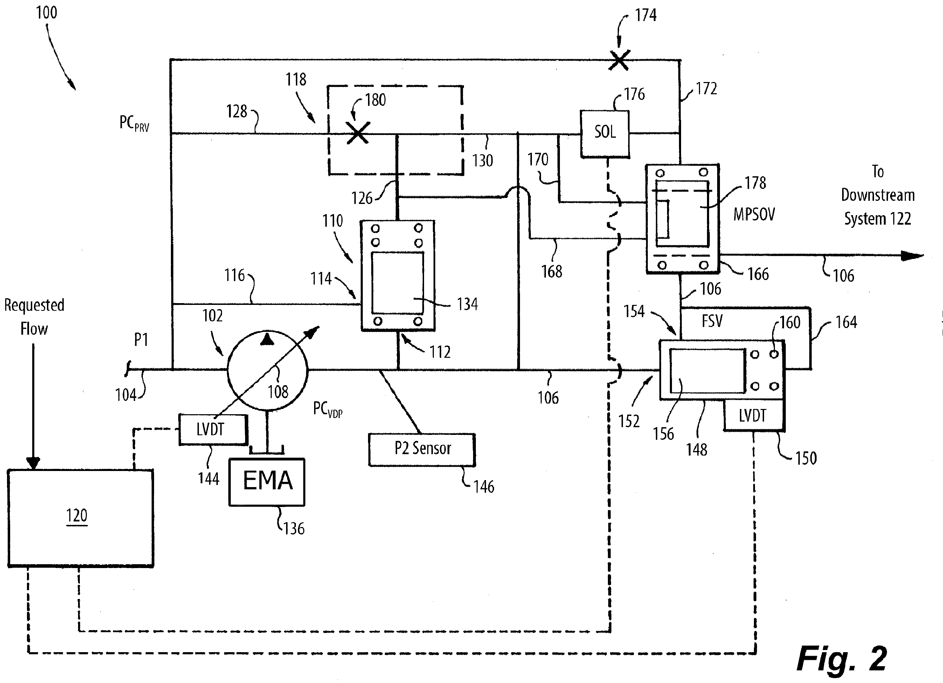

Reference will now be made to the drawings wherein like reference numerals identify similar structural features or aspects of the subject disclosure. For purposes of explanation and illustration, and not limitation, a partial view of an embodiment of a system in accordance with the disclosure is shown in Fig. 1 and is designated generally by reference character 100. Other embodiments of systems in accordance with the disclosure, or aspects thereof, are provided in Fig. 2, as will be described. The systems and methods described herein can be used to provide direct electrical actuation control for variable displacement pumps, such as for use in metering/supplying fuel to gas generators in aircraft engines.

Utilizing a variable displacement pump with direct pump displacement control via an electro-mechanical actuator and a pressure sensor, a pump position can be set to maintain a pressure and a flow schedule to support flow demands for a gas generator or augmenter. Low end pump flows can either be handled by the pump itself (e.g. wherein pump minimum flow is less than the minimum fuel delivery requirement) or via an actively controlled bypass valve (e.g. where the pump's minimum flow greater than the minimum fuel delivery requirement). A flow sensing valve can be used to determine flow sent to gas generator or augmenter and a shutoff valve is used provide fast shut-off response. As described below, the shutoff valve can also trigger a bypass valve for pump recirculation.

The system 100 includes a variable displacement pump (VDP) 102 in fluid communication with an inlet line 104 and with an outlet line 106. The VDP 102 includes a variable displacement mechanism 108 configured to vary pressure to the outlet line 106. The variable displacement mechanism 108 is connected to be actuated directly by an electromechanical actuator (EMA) 136 for direct electrical/mechanical control of the variable displacement mechanism 108, i.e. without the need for any hydraulic component of actuation for the variable displacement mechanism 108 as opposed, e.g., to the use of hydraulics as in electrohydraulic servo valve (EHSV) actuators.

A bypass valve (BPV) 110 includes a BPV inlet 112 in fluid communication with the outlet line 106, and a BPV outlet 114 in fluid communication with a bypass line 116 that feeds into the inlet line 104 upstream of the VDP 102. An actuator 118 is operatively connected to control the BPV 110 to vary flow from the BPV inlet 112 to the bypass line 116. A flow sensing valve (FSV) 148 is connected in the outlet line 106. The FSV 148 includes a sensor 150, such as a linear variable differential transformer (LVDT), configured to generate sensor data indicative of flow out of the outlet line 106 to the downstream system 122 supplied by the outlet line 106.

A controller 120 i is operatively connected to the controller 120 for active control of the EMA 136 to actuate the variable displacement mechanism 108 to control flow through the VDP 102. The controller 120 is operatively connected to the actuator 118 to control recirculation flow passed through the BPV 110 based on the sensor data and based on a predetermined low threshold of flow through the VDP 102. The downstream system 122 can be a combustor, augmenter, or other gas generator of a gas turbine engine, for example. The low threshold of flow of the VDP 104 can be the threshold below which the VDP 104 cannot self-lubricate, or other design requirements for low or minimum flow. The BPV outlet 114 can be the only outlet of the BPV 110 so all flow through the BPV 110 from the BPV inlet 112 is supplied to the BPV outlet 114.

The controller 120 is configured, e.g. including analog circuitry, digital logic, and/or machine readable instructions, to control the BPV 110 to maintain a baseline flow through the BPV 110 under a first condition wherein requested flow from the downstream system 122 is above than the predetermined low threshold. The controller 122 is configured to control the BPV 110 to increase the flow through the BPV 110 above the baseline flow for a second flow condition wherein requested flow from the downstream system 122 is at or below the predetermined low threshold.

The actuator 118 includes a first electrohydraulic servo valve (EHSV) 124 that is connected in fluid communication with the BPV 110 by a first control line 126. The first EHSV 124 is connected in fluid communication with both the inlet line 104, by way of the recirculation line 116, and with the outlet line 106 through respective connection lines 128, 130. The first EHSV 124 is operatively connected to the controller 120 for active control of the first EHSV 124 to actuate the BPV 110.

A minimum pressure shutoff valve (MPSOV) 166 is connected in fluid communication with the outlet line 106 downstream of the FSV 148, configured to block flow through the outlet line 106 for shutoff of flow from the outlet line to the downstream system 122, e.g. to shut off flow to fuel injectors or the like. The MPSOV 166 is connected in fluid communication with the BPV 110 for triggering recirculation through the BPV 110.

A first MPSOV line 168 connects the MPSOV 166 to the first control line 126 for fluid communication. A second MPSOV line 170 connects the MPSOV 166 to the inlet line 104 for fluid communication. An MPSOV control line 172 connects the MPSOV 166 in fluid communication with the inlet line 104 through a fixed throttle or orifice 174. A solenoid valve (SOL) 176 is connected in fluid communication with the outlet line 106 and with the MPSOV control line 172 for actuating the MPSOV 166 between first and second states. The SOL 176 is connected to the controller 120 for control of the MPSOV 166 by the controller 122. The MPSOV 166 includes an MPSOV valve member 178 with a first position in the MPSOV 166 that connects the first and second MPSOV lines 168, 170 in fluid communication and blocks flow through the outlet line to shut off flow to the downstream systems. The second position of the valve member 178 is schematically indicated in Fig. 2 with dashed lines inside the MPSOV 166. In this second position, the valve member 178 blocks fluid communication of the first and second MPSOV lines 168, 170 and allows flow though the outlet line 106 to the downstream system 122.

In the first position, the MPSOV 166 connects BPV 110 to inlet line 104. When a downstream system 122 such as a gas generator is shut down but the VDP 102 is still spinning, e.g. when a gas turbine engine is shut off but is still spooling down in speed, the VDP 102 needs a safe means to recirculate flow until the pump speed has reached 0 rpm. A high pressure relief valve can be included for to protection from overpressurization, but it is better for the VDP 102 not to go into high pressure relief every time downstream flow is turned off, since that would impose excess wear and tear on the VDP 102.

While the MPSOV 166 has an infinite number of positions across its stroke, the timing of the line 168 relative to valve stroke is such that as soon as the MPSOV 166 beings to open, the outlet port to the outlet line 106 (leading to the downstream system 122) is shut off. This allows the EHSV 124 to then control the BPV 110 to modulate bypass flow. When the port is open, the flow capacity of the line is greater than the EHSV 124 can provide to the circuit thereby rendering the EHSV 124 unable to raise the pressure above the inlet pressure plus some minor amount for plumbing losses. So the BPV 110 is controlled by the balance of pump outlet pressure versus the sum of inlet pressure and spring force in the BPV 110. So at almost all positions above the closed position of the MPSOV 166, the EHSV 124 can open the BPV 110 to cause a flow split between BPV 110 and the FSV 148. This can be utilized any time demanded flow is less than pump minimum flow or any time engine power is rapidly cut to stabilize the demand flow while the fuel system readjusts, for example.

A first position sensor 132, such as a linear variable differential transformer (LVDT), is operatively connected to the BPV 110 to provide sensor output indicative of position of a valve member 134 of the BPV 110 within the BPV 110. The first position sensor 132 is operatively connect the controller 120 to provide feedback for controlling the BPV 110. A second position sensor 144, such as an LVDT, is operatively connected to the variable displacement mechanism 108 to provide sensor output indicative of position of the variable displacement mechanism 108, wherein the second position sensor 144 is operatively connect the controller 120 to provide feedback for controlling the variable displacement mechanism 108 directly with the EMA 136.

A pressure sensor 146 is operatively connected to the outlet line 106 to generate sensor output indicative of pressure in the outlet line 106. The pressure sensor 146 is operatively connected to the controller 120 for active control of the variable displacement mechanism 108 and/or of the BPV 110 based on pressure in the outlet line 106. The sensor output of sensor 150 is indicative of flow demanded by the downstream system 122. The controller 122 is configured to control position of the valve member 134 of the BPV 110 to maintain bypass flow through the BPV in the second condition, i.e. when recirculation through the BPV 110 is needed because flow demanded by the downstream system 122 drops below the predetermined low threshold for flow through the VDP 102. In this second condition, the controller 120 governs the bypass flow through the BPV 110 according to

$$BF = PF - DSFD$$

wherein BF is flow through the BPV 110, PF is flow through the VDP 102 (e.g. as indicated by sensors 144 and/or 146), and DSFD is flow demanded by the downstream system 122 as indicated by the sensor output of the sensor 150. In the first condition, e.g. when flow demanded by the downstream system 122 is at or above the predetermined low threshold for flow through the VDP 112, the controller 120 controls the BPV 110 to recirculate flow from the outlet line 106 to the inlet line 102 at a constant base recirculation rate. The base recirculation rate can be zero recirculation flow.

The FSV 148 includes an FSV inlet 152, an FSV outlet 154, and a valve member 156 that moves inside the FSV 148 based on how much pressure is acting on the valve member 156. A biasing member 158 biases the valve member 160 in a first direction, e.g. the biasing member 160 can bias the valve member to the left as oriented in Fig. 1. Pressure of flow through the FSV 148 from the FSV inlet 152 to the FSV outlet 154 biases the valve member 156 in a second direction opposite the first direction, e.g. to the right as oriented in Fig. 1. The sensor 150 includes a position sensor operatively connected to monitor position of the valve member 156 in the FSV 148 to generate the sensor data indicative of that position. The FSV 148 includes a pressure port 162 on a side of the valve member 156 opposite from the FSV inlet 152 and the FSV outlet 154. A pressure line 164 connects the FSV outlet 154 in fluid communication with the pressure port 162. The pressure port 162 of the FSV 148 connects the backside of the FSV 148 to the downstream pressure in the outlet line 106, via the pressure line 164. The pressure balance on the FSV 148 is pump outlet pressure versus the sum of pressure from FSV outlet pressure (via pressure line 164) and from the spring force from biasing member 160. The biasing member 160 creates a constant pressure drop across the FSV 148 to allow for the controller 120 to calculate flow across the FSV 120.

Fig. 1 shows system 100 configured for active control of the BPV 110. With reference now to Fig. 2, system 100 is shown configured for passive control of the BPV 110, where the other components similarly numbered are similar in operation to those described above with respect to Fig. 1 except as noted in the following with reference to Fig. 2.

With reference now to Fig. 2, the BPV control line 126 connects the BPV 110 in fluid communication with the inlet line 104 through a first fixed throttle or orifice 180. The actuator 118 in this case includes the BPV control line 126 and the fixed throttle or orifice 180, and the first MPSOV line 168 that connects the MPSOV in fluid communication with the BPV control line 126 for passive control of the BPV 110. The second MPSOV line 170 connects the MPSOV 166 to the outlet line 106 for fluid communication. The MPSOV control line 172 connects the MPSOV 166 in fluid communication with the inlet line 104 through the second fixed throttle or orifice 174. The SOL 176 is connected in fluid communication with the outlet line 106 and with the MPSOV control line 172 for actuating the MPSOV 166 between first and second states. The MPSOV 166 in this configuration includes an MPSOV valve member 178 with a first position in the MPSOV 166 that disconnects the first and second MPSOV lines 168, 170 from being in fluid communication to allow bypass flow through the BPV 110 and to block flow through the outlet line 106 to shut off flow to the downstream systems 122. The first position of the valve member 178 is indicated in Fig. 2 with the dashed lines inside the MPSOV 166. The valve member 178 as configured in Fig. 2 has a second position that connects the first and second MPSOV lines 168, 170 in fluid communication to shutoff bypass flow through the BPV 110 and to allow flow though the outlet line 106, e.g. to supply the downstream systems 122.

The low threshold flow for the VDP 102 can either be handled by the VDP 102 itself, e.g. for applications where the VDP low flow threshold or requirement is less than the designed low threshold for fuel delivery to the downstream system 122, or via an actively controlled BPV 110, e.g. in applications where the low flow requirement or threshold for the VDP 102 is greater than the low flow threshold designed for fuel delivery to the downstream system 122. An FSV 148 can be used to determine flow sent to downstream systems 122, e.g. gas generators, augmenters, or the like. An MPSOV 178 is used to set the low pressure threshold for shutoff and to provide fast shut-off response. The MPSOV 178 can also trigger actuation of the BPV 110 for pump recirculation.

Systems and methods as disclosed herein provide various potential benefits including the following. There can be a reduction in valve count. The metering system can be faster than legacy systems, e.g. where a metering valve controls pressure control valve (PCV) which controls pump displacement or the like. There can be a reduced horsepower extraction at low power conditions, e.g. there need be no minimum pressurization required for pump control. Systems and methods as disclosed herein can reduce fuel system weight and pump system heat rejection, allowing aircraft to carry more fuel and increase aircraft heat rejection into the fuel.

The methods and systems of the present disclosure, as described above and shown in the drawings, provide direct electrical actuation control for variable displacement pumps, such as for use in metering/supplying fuel to gas generators in aircraft engines. While the apparatus and methods of the subject disclosure have been shown and described with reference to preferred embodiments, those skilled in the art will readily appreciate that changes and/or modifications may be made thereto without departing from the scope of the invention as defined by the claims.

**Claims**

1. A system comprising:

   a variable displacement pump, VDP, (102) in fluid communication with an inlet line and with an outlet line, wherein the VDP includes a variable displacement mechanism configured to vary pressure to the outlet line;
   an electromechanical actuator, EMA, (136) operatively connected to actuate the variable displacement mechanism;
   a bypass valve, BPV, (110) including a BPV inlet (112) in fluid communication with the outlet line, and a BPV outlet (114) in fluid communication with a bypass line that feeds into the inlet line upstream of the VDP;
   an actuator (118) operatively connected to control the BPV to vary flow from the BPV inlet to the bypass line;
   a flow sensing valve, FSV, (148) connected in the outlet line, wherein the FSV includes a sensor (150) configured to generate sensor data indicative of flow out of the outlet line;
   a controller (120) operatively connected:

   to the EMA (136) to control the variable displacement mechanism; and
   to the actuator (118) to control recirculation flow passed through the BPV based on the sensor data and based on a predetermined low threshold of flow through the VDP;

   a minimum pressure shutoff valve, MPSOV, (166) connected in fluid communication with the outlet line, configured to block flow through the outlet line for shutoff, wherein the MPSOV is connected in fluid communication with the BPV to control the BPV to recirculate flow from the outlet line to an input line of the VDP in the event of the sensor feedback dropping below a predetermined low threshold of flow through the VDP, wherein the MPSOV is connected to selectively shut off flow in the outlet line;
   wherein controlling the BPV to recirculate flow includes governing the bypass flow through the BPV according to

$$BF = PF\text{-}DSFD$$

   wherein BF is flow through the BPV, PF is flow through the VDP, and DSFD is flow demanded by the downstream system supplied by the outlet line as indicated by the sensor feedback.

2. The system as recited in claim 1, wherein the controller is configured to:

   control the BPV to maintain a baseline flow through the BPV under a first condition wherein requested flow from the downstream system is above than the predetermined low threshold, and
   control the BPV to increase the flow through the BPV above the baseline flow for a second flow condition wherein requested flow from the downstream system is at or below the predetermined low threshold.

3. The system as recited in claim 2, further comprising:

   a first electrohydraulic servo valve, EHSV, (124) connected in fluid communication with the BPV by a first control line (126), wherein the first EHSV is connected in fluid communication with both the inlet line and with the outlet line through respective connection lines, and wherein the first EHSV is operatively connected to the controller for active control of the first EHSV to actuate the BPV;
   a first MPSOV line (168) connecting the MPSOV to the first control line for fluid communication;
   a second MPSOV line (170) connecting the MPSOV to the inlet line for fluid communication;
   an MPSOV control line (172) that connects the MPSOV in fluid communication with the inlet line through a fixed throttle; and
   a solenoid valve, SOL, (176) connected in fluid communication with the outlet line and with the MPSOV control line for actuating the MPSOV between first and second states,
   wherein the MPSOV includes an MPSOV valve member (178) with a first position in the MPSOV that connects the first and second MPSOV lines in fluid communication and blocks flow through the outlet line, and a second position that blocks fluid communication of the first and second MPSOV lines and allows flow though the outlet line, and optionally wherein a first position sensor (132) is operatively connected to the BPV to provide sensor output indicative of position of a valve member of the BPV, wherein the first position sensor is operatively connected to the controller to provide feedback for controlling the BPV.

4. The system as recited in claim 3, further comprising a second EHSV connected in fluid communication with the variable displacement mechanism by a second control line for control of flow through the VDP, wherein the second EHSV is connected in fluid communication with both the inlet line and with the outlet line through respective connection lines, and wherein the second EHSV is operatively connected to the controller for active control of the second EHSV to actuate the variable displacement mechanism; and optionally

wherein a second position sensor (144) is operatively connected to the variable displacement mechanism to provide sensor output indicative of position of the variable displacement mechanism, wherein the second position sensor is operatively connected to the controller to provide feedback for controlling the variable displacement mechanism.

5. The system as recited in claim 4, further comprising a pressure sensor (146) operatively connected to the outlet line to generate sensor output indicative of pressure in the outlet line, wherein the pressure sensor is operatively connected to the controller for active control of the variable displacement mechanism and/or of the BPV based on pressure in the outlet line, and optionally wherein the sensor output is indicative of flow demanded by the downstream system supplied by the outlet line.

6. The system as recited in claim 1, wherein the FSV includes an FSV inlet (152), an FSV outlet (154), and a valve member (178), wherein a biasing member (160) biases the valve member in a first direction, and wherein pressure of flow through the FSV from the FSV inlet to the FSV outlet biases the valve member in a second direction opposite the first direction.

7. The system as recited in claim 6, wherein the sensor includes a position sensor operatively connected to monitor position of the valve member in the FSV to generate the sensor data; and optionally wherein the FSV includes a pressure port (162) on a side of the valve member opposite from the FSV inlet and the FSV outlet, wherein a pressure line connects the FSV outlet in fluid communication with the pressure port.

8. The system as recited in claim 7, further comprising:

a BPV control line (126) connecting the BPV in fluid communication with the inlet line through a first fixed throttle;
a first MPSOV line (168) connecting the MPSOV in fluid communication with the BPV control line;
a second MPSOV line (170) connecting the MPSOV to the outlet line for fluid communication;
an MPSOV control line (172) that connects the MPSOV in fluid communication with the inlet line through a fixed throttle; and
a solenoid valve, SOL, (176) connected in fluid communication with the outlet line and with the MPSOV control line for actuating the MPSOV between first and second states, wherein the MPSOV includes an MPSOV valve member (178) with a first position in the MPSOV that disconnects the first and second MPSOV lines from being in fluid communication and blocks flow through the outlet line, and a second position that connects the first and second MPSOV lines in fluid communication and allows flow though the outlet line.

9. A method comprising:

controlling a variable displacement pump, VDP, (102) by actuating a variable displacement mechanism of the VDP with an electromechanical actuator, EMA (136);
receiving sensor feedback from a flow sensing valve, FSV, (148) indicative of flow demanded by a downstream system supplied from an outlet line of the variable displacement pump, VDP; and
controlling a bypass valve, BPV, (110) with a minimum pressure shutoff valve, MPSOV, (166) to recirculate flow from the outlet line to an input line of the VDP in the event of the sensor feedback dropping below a predetermined low threshold of flow through the VDP, wherein the MPSOV is connected to selectively shut off flow in the outlet line;
wherein controlling the BPV to recirculate flow includes governing the bypass flow through the BPV according to

$$BF = PF-DSFD$$

wherein BF is flow through the BPV, PF is flow through the VDP, and DSFD is flow demanded by the downstream system supplied by the outlet line as indicated by the sensor feedback.

10. The method as recited in claim 9, further comprising controlling the BPV to recirculate flow from the outlet line to the inlet line at a constant base recirculation rate in the event of flow demanded by the downstream system being at or above the predetermined low threshold of flow through the VDP, and optionally wherein the base recirculation rate is zero recirculation flow.

11. The method as recited in claim 9, further comprising receiving pressure data from a pressure sensor (146) in the outlet line, wherein controlling the BPV to recirculate flow includes controlling the BPV based at least in part on the pressure data.

12. The method as recited in claim 9, further comprising receiving data from a sensor indicative of position of a valve member of the BPV, wherein controlling the BPV includes controlling the BPV based on position of the valve member.

**Patentansprüche**

1. System, umfassend:

   eine Verstellpumpe, VDP, (102) in Fluidverbindung mit einer Einlassleitung und mit einer Auslassleitung, wobei die VDP einen Verstellmechanismus beinhaltet, der zum Variieren des Drucks zu der Auslassleitung konfiguriert ist; einen elektromechanischen Aktor, EMA, (136) der zum Betätigen des Verstellmechanismus wirkverbunden ist; ein Bypassventil, BPV, (110), das einen BPV-Einlass (112) in Fluidverbindung mit der Auslassleitung und einen BPV-Auslass (114) in Fluidverbindung mit einer Bypassleitung beinhaltet, die der VDP vorgeschaltet in die Einlassleitung eingeführt ist; einen Aktor (118), der zum Variieren von Durchfluss von dem BPV-Einlass zu der Bypass-Leitung mit dem BPV wirkverbunden ist; ein in der Auslassleitung verbundenes Durchflusserfassungsventil, FSV, (148), wobei das FSV einen Sensor (150) beinhaltet, der zum Erzeugen von Sensordaten konfiguriert ist, die Durchfluss aus der Auslassleitung angeben; einen Regler (120), der wirkverbunden ist:

   mit dem EMA (136), um den Verstellmechanismus zu regeln; und mit dem Aktor (118), um durch das BPV passierten Rezirkulationsdurchfluss basierend auf den Sensordaten und basierend auf einem vorbestimmten unteren Schwellenwert für Durchfluss durch die VDP zu regeln; ein Mindestdruckabsperrventil, MPSOV, (166), das in Fluidverbindung mit der Auslassleitung verbunden ist und zum Blockieren von Durchfluss durch die Auslassleitung zum Absperren konfiguriert ist, wobei das MPSOV in Fluidverbindung mit dem BPV verbunden ist, um das BPV zum Rezirkulieren von Durchfluss von der Auslassleitung zu einer Einspeiseleitung der VDP zu regeln, falls die Sensorrückmeldungen unter einen vorbestimmten unteren Schwellenwert für Durchflusses durch den VDP fallen, wobei das MPSOV zum selektiven Absperren von Durchfluss in der Auslassleitung verbunden ist; wobei Regeln des BPV zum Rezirkulieren von Durchfluss ein Regeln des Bypassdurchflusses durch das BPV gemäß Folgendem beinhaltet

   $$BF = PF - DSFD$$

   wobei BF Durchfluss durch das BPV ist, PF Durchfluss durch die VDP ist und DSFD Durchfluss ist, der durch das von der Auslassleitung versorgte nachgeschaltete System angefordert wird, wie durch die Sensorrückmeldungen angegeben.

2. System nach Anspruch 1, wobei der Regler zu Folgendem konfiguriert ist:

   Regeln des BPV, um einen Basisdurchfluss durch das BPV unter einer ersten Bedingung aufrechtzuerhalten, wobei geforderter Durchfluss von dem nachgeschalteten System über dem vorbestimmten unteren Schwellenwert liegt, und Regeln des BPV, um den Durchfluss durch das BPV für eine zweite Durchflussbedingung über den Basisdurchfluss zu erhöhen, wobei der geforderte Durchfluss von dem nachgeschalteten System bei oder unter dem vorbestimmten unteren Schwellenwert liegt.

3. System nach Anspruch 2, ferner umfassend:

   ein erstes elektrohydraulisches Servoventil, EHSV, (124) das über eine erste Regelleitung (126) in Fluidkommunikation mit dem BPV verbunden ist, wobei das erste EHSV durch jeweilige Verbindungsleitungen in Fluidkommunikation sowohl mit der Einlassleitung als auch mit der Auslassleitung verbunden ist und wobei das erste EHSV zur aktiven Regelung des ersten EHSV mit dem Regler wirkverbunden ist, um das BPV zu betätigen; eine erste MPSOV-Leitung (168), die das MPSOV zur Fluidkommunikation mit der ersten Regelleitung verbindet; eine zweite MPSOV-Leitung (170), die das MPSOV zur Fluidkommunikation mit der Einlassleitung verbindet; eine MPSOV-Regelleitung (172), die das MPSOV über eine feste Drosselklappe in Fluidverbindung mit der Einlassleitung verbindet; und ein Magnetventil, SOL, (176), das mit der Auslassleitung und mit der MPSOV-Regelleitung zum Betätigen des MPSOV zwischen einem ersten und zweiten Zustand in Fluidverbindung verbunden ist, wobei das MPSOV ein MPSOV-Ventilelement (178) mit einer ersten Position in dem MPSOV, in der die erste und zweite MPSOV-Leitung in Fluidverbindung verbunden sind und Durchfluss durch die Auslassleitung blockiert ist, und einer zweiten Position beinhaltet, in der die Fluidverbindung der ersten und zweiten MPSOV-Leitung blockiert ist und Durchfluss durch die Auslassleitung möglich ist, und wobei optional ein erster Positionssensor (132) mit dem BPV wirkverbunden ist, um Sensorausgaben bereitzustellen, die eine Position eines Ventilelements des BPV angeben, wobei der erste Positionssensor mit der Regelung wirkverbunden ist, um Rückmeldungen zur Regelung des BPV bereitzustellen.

4. System nach Anspruch 3, ferner umfassend ein zweites EHSV, das über eine zweite Regelleitung zur Regelung von Durchfluss durch die VDP mit dem Verstellmechanismus in Fluidverbindung verbunden ist, wobei das zweite EHSV sowohl mit der Einlassleitung als auch mit der Auslassleitung durch jeweilige Verbindungsleitungen in Fluidverbindung verbunden ist und wobei das zweite EHSV mit der Regelung zur aktiven Regelung des zweiten EHSV wirkverbunden ist, um den Verstellmechanismus zu betätigen; und optional
wobei ein zweiter Positionssensor (144) mit dem Verstellmechanismus wirkverbunden ist, um Sensorausgaben bereitzustellen, die die Position des Verstellmechanismus angeben, wobei der zweite Positionssensor mit der Regelung wirkverbunden ist, um Rückmeldungen zur Regelung des Verstellmechanismus bereitzustellen.

5. System nach Anspruch 4, ferner umfassend einen Drucksensor (146), der mit der Auslassleitung wirkverbunden ist, um Sensorausgaben zu erzeugen, die Druck in der Auslassleitung angeben, wobei der Drucksensor zur aktiven Regelung des Verstellmechanismus und/oder des BPV basierend auf Druck in der Auslassleitung mit der Regelung wirkverbunden ist und wobei die Sensorausgaben optional Durchfluss angeben, der durch das durch die Auslassleitung versorgte nachgeschaltete System angefordert wird.

6. System nach Anspruch 1, wobei das FSV einen FSV-Einlass (152), einen FSV-Auslass (154) und ein Ventilelement (178) beinhaltet, wobei ein Vorspannelement (160) das Ventilelement in eine erste Richtung vorspannt und wobei Druck von Durchfluss durch das FSV von dem FSV-Einlass zu dem FSV-Auslass das Ventilelement in eine zweite, der ersten Richtung entgegengesetzte Richtung vorspannt.

7. System nach Anspruch 6, wobei der Sensor einen Positionssensor beinhaltet, der zum Überwachen einer Position des Ventilelements in dem FSV wirkverbunden ist, um die Sensordaten zu erzeugen; und optional
wobei das FSV einen Druckanschluss (162) auf einer Seite des Ventilelements gegenüber dem FSV-Einlass und dem FSV-Auslass beinhaltet, wobei eine Druckleitung den FSV-Auslass in Fluidverbindung mit dem Druckanschluss verbindet.

8. System nach Anspruch 7, ferner umfassend:

eine BPV-Regelleitung (126), die das BPV über eine erste feste Drosselklappe in Fluidverbindung mit der Einlassleitung verbindet;
eine erste MPSOV-Leitung (168), die das MPSOV mit der BPV-Regelleitung in Fluidkommunikation verbindet;
eine zweite MPSOV-Leitung (170), die das MPSOV zur Fluidkommunikation mit der Auslassleitung verbindet;
eine MPSOV-Regelleitung (172), die das MPSOV über eine feste Drosselklappe in Fluidverbindung mit der Einlassleitung verbindet; und
ein Magnetventil, SOL, (176), das mit der Auslassleitung und mit der MPSOV-Regelleitung zum Betätigen des MPSOV zwischen einem ersten und zweiten Zustand in Fluidverbindung verbunden ist,
wobei das MPSOV ein MPSOV-Ventilelement (178) mit einer ersten Position in dem MPSOV, in der die Fluidverbindung der ersten und zweiten MPSOV-Leitung getrennt ist und Durchfluss durch die Auslassleitung blockiert ist, und einer zweiten Position beinhaltet, in der die erste und zweite MPSOV-Leitung in Fluidverbindung verbunden sind und Durchfluss durch die Auslassleitung möglich ist.

9. Verfahren, umfassend:

Regeln einer Verstellpumpe, VDP, (102) durch Betätigen eines Verstellmechanismus der VDP mit einem elektromechanischen Aktor, EMA, (136);
Empfangen von Sensorrückmeldungen von einem Durchflusserfassungsventil, FSV, (148), die Durchfluss angeben, der durch ein durch eine Auslassleitung der Verstellpumpe, VDP, versorgtes nachgeschaltetes System angefordert wird; und
Regeln eines Bypassventils, BPV, (110) mit einem Mindestdruckabsperrventil, MPSOV, (166), um Durchfluss von der Auslassleitung zu einer Einspeiseleitung der VDP zu rezirkulieren, falls die Sensorrückmeldungen unter einen vorbestimmten unteren Schwellenwert für Durchflusses durch den VDP fallen, wobei das MPSOV zum selektiven Absperren von Durchfluss in der Auslassleitung verbunden ist;
wobei Regeln des BPV zum Rezirkulieren von Durchfluss ein Regeln des Bypassdurchflusses durch das BPV gemäß Folgendem beinhaltet

$$BF = PF-DSFD$$

wobei BF Durchfluss durch das BPV ist, PF Durchfluss durch die VDP ist und DSFD Durchfluss ist, der durch das von der Auslassleitung versorgte nachgeschaltete System angefordert wird, wie durch die Sensorrückmeldungen angegeben.

10. Verfahren nach Anspruch 9, ferner umfassend Regeln des BPV, um Durchfluss von der Auslassleitung zu der Einlassleitung mit einer konstanten Basisrezirkulationsrate zu rezirkulieren, falls von dem nachgeschalteten System angeforderter Durchfluss bei oder über dem vorbestimmten unteren Schwellenwert von Durchfluss durch das VDP liegt und optional

wobei die Basisrezirkulationsrate ein Nullrezirkulationsdurchfluss ist.

11. Verfahren nach Anspruch 9, ferner umfassend Empfangen von Druckdaten von einem Drucksensor (146) in der Auslassleitung, wobei Regeln des BPV zum Rezirkulieren von Durchfluss Regeln des BPV mindest teilweise basierend auf den Druckdaten beinhaltet.

12. Verfahren nach Anspruch 9, ferner umfassend Empfangen von Daten von einem Sensor, die eine Position eines Ventilelements des BPV angeben, wobei Regeln des BPV Regeln des BPV basierend auf einer Position des Ventilelements beinhaltet.

## Revendications

1. Système comprenant :

une pompe à cylindrée variable, VDP (102) en communication fluidique avec une conduite d'entrée et avec une conduite de sortie, dans lequel la VDP comporte un mécanisme à cylindrée variable configuré pour faire varier la pression vers la conduite de sortie ;
un actionneur électromécanique, EMA (136), raccordé de manière fonctionnelle pour actionner le mécanisme à cylindrée variable ;
une vanne de dérivation, BPV, (110) comportant une entrée de BPV (112) en communication fluidique avec la conduite de sortie, et une sortie de BPV (114) en communication fluidique avec une conduite de dérivation qui alimente la conduite d'entrée en amont de la VDP ;
un actionneur (118) raccordé de manière fonctionnelle pour commander la BPV afin de faire varier l'écoulement de l'entrée de BPV vers la conduite de dérivation ;
une vanne de détection de débit, FSV (148), raccordée dans la conduite de sortie, dans lequel la FSV comporte un capteur (150) configuré pour générer des données de capteur indiquant un écoulement sortant de la conduite de sortie ;
un dispositif de commande (120) raccordé de manière fonctionnelle :

à l'EMA (136) pour commander le mécanisme à cylindrée variable ; et
à l'actionneur (118) pour commander l'écoulement de recirculation passant à travers la BPV en fonction des données de capteur et en fonction d'un seuil bas prédéterminé d'écoulement à travers la VDP ;
une vanne d'arrêt de pression minimale, MPSOV, (166) raccordée en communication fluidique avec la conduite de sortie, configurée pour bloquer l'écoulement à travers la conduite de sortie pour l'arrêt, dans lequel la MPSOV est raccordée en communication fluidique avec la BPV pour commander la BPV afin de recirculer l'écoulement de la conduite de sortie vers une conduite d'entrée de la VDP dans le cas où le retour de capteur tombe en dessous d'un seuil bas prédéterminé d'écoulement à travers la VDP, dans lequel la MPSOV est raccordée pour arrêter sélectivement l'écoulement dans la conduite de sortie ;
dans lequel la commande de la BPV pour recirculer l'écoulement comporte la régulation de l'écoulement de dérivation à travers la BPV selon

$$BF = PF-DSFD$$

dans lequel BF est un écoulement à travers la BPV, PF est un écoulement à travers la VDP, et DSFD est un écoulement demandé par le système en aval alimenté par la conduite de sortie comme indiqué par le retour de capteur.

2. Système selon la revendication 1, dans lequel le dispositif de commande est configuré pour :

commander la BPV afin de maintenir un écoulement de ligne de base à travers la BPV lors d'une première condition dans lequel l'écoulement demandé par le système en aval est supérieur au seuil bas prédéterminé ; et
commander la BPV afin d'augmenter l'écoulement à travers la BPV au-delà de l'écoulement de ligne de base lors d'une seconde condition d'écoulement dans lequel l'écoulement demandé par le système en aval est égal ou inférieur au seuil bas prédéterminé.

**3.** Système selon la revendication 2, comprenant en outre :

une première servovalve électrohydraulique, EHSV (124) raccordée en communication fluidique avec la BPV par une première conduite de commande (126), dans lequel la première EHSV est raccordée en communication fluidique à la fois avec la conduite d'entrée et avec la conduite de sortie par des conduites de raccordement respectives, et dans lequel la première EHSV est raccordée de manière fonctionnelle au dispositif de commande pour la commande active de la première EHSV afin d'actionner la BPV ;

une seconde conduite de MPSOV (168) raccordant la MPSOV à la première conduite de commande pour la communication fluidique ;

une seconde conduite de MPSOV (170) raccordant la MPSOV à la conduite d'entrée pour la communication fluidique ;

une conduite de commande de MPSOV (172) qui raccorde la MPSOV en communication fluidique avec la conduite d'entrée par un étrangleur fixe ; et

une électrovanne, SOL, (176) raccordée en communication fluidique avec la conduite de sortie et avec la conduite de commande de MPSOV pour actionner la MPSOV entre des premier et second états,

dans lequel la MPSOV comporte un élément de vanne MPSOV (178) avec une première position dans la MPSOV qui raccorde les première et seconde conduites de MPSOV en communication fluidique et bloque l'écoulement à travers la conduite de sortie, et une second position qui bloque la communication fluidique des première et seconde conduites de MPSOV et permet l'écoulement à travers la conduite de sortie, et éventuellement dans lequel un premier capteur de position (132) est raccordé de manière fonctionnelle à la BPV pour fournir une sortie de capteur indiquant une position d'un élément de vanne de la BPV, dans lequel le premier capteur de position est raccordé de manière fonctionnelle au dispositif de commande pour fournir un retour pour la commande de la BPV.

**4.** Système selon la revendication 3, comprenant en outre une seconde EHSV raccordée en communication fluidique avec le mécanisme à cylindrée variable par une seconde conduite de commande pour la commander de l'écoulement à travers la VDP ; dans lequel la seconde EHSV est raccordée en communication fluidique à la fois avec la conduite d'entrée et avec la conduite de sortie par des conduites de liaison respectives, et dans lequel la seconde EHSV est raccordée de manière fonctionnelle au dispositif de commande pour la commande active de la seconde EHSV afin d'actionner le mécanisme à cylindrée variable ; et éventuellement dans lequel un second capteur de position (144) est raccordé de manière fonctionnelle au mécanisme à cylindrée variable pour fournir une sortie de capteur indiquant une position du mécanisme à cylindrée variable, dans lequel le second capteur de position est raccordé de manière fonctionnelle au dispositif de commande pour fournir un retour visant à commander le mécanisme à cylindrée variable.

**5.** Système selon la revendication 4, comprenant en outre un capteur de pression (146) raccordé de manière fonctionnelle à la conduite de sortie pour générer une sortie de capteur indiquant une pression dans la conduite de sortie, dans lequel le capteur de pression est raccordé de manière fonctionnelle au dispositif de commande pour la commande active du mécanisme à cylindrée variable et/ou de la BPV en fonction de la pression dans la conduite de sortie, et éventuellement dans lequel la sortie de capteur indiquant l'écoulement demandé par le système en aval alimenté par la conduite de sortie.

**6.** Système selon la revendication 1, dans lequel la FSV comporte une entrée de FSV (152), une sortie de FSV (154) et un élément de vanne (178), dans lequel un élément de rappel (160) rappelle l'élément de vanne dans une première direction, et dans lequel une pression d'écoulement à travers la FSV de l'entrée de FSV vers la sortie de FSV rappelle l'élément de vanne dans une seconde direction opposée à la première direction.

**7.** Système selon la revendication 6, dans lequel le capteur comporte un capteur de position raccordé de manière fonctionnelle pour surveiller une position de l'élément de vanne dans la FSV afin de générer les données de capteur ; et éventuellement dans lequel la FSV comporte un orifice de pression (162) sur un côté de l'élément de vanne opposé à l'entrée de FSV et à la sortie de FSV, dans lequel une conduite de pression raccorde la sortie de FSV en communication fluidique avec l'orifice de pression.

**8.** Système selon la revendication 7, comprenant, en outre :

une conduite de commande de BPV (126) raccordant la BPV en communication fluidique avec la conduite d'entrée par un premier étrangleur fixe ;

une première conduite de MPSOV (168) raccordant la MPSOV en communication fluidique avec la conduite de commande de BPV ;

une seconde conduite de MPSOV (170) raccordant la MPSOV à la conduite de sortie pour la communication fluidique ;

une conduite de commande de MPSOV (172) qui raccorde la MPSOV en communication fluidique avec la conduite d'entrée par un étrangleur fixe ; et

une électrovanne, SOL, (176) raccordée en communication fluidique avec la conduite de sortie et avec la conduite de commande de MPSOV pour actionner la MPSOV entre des premier et second états,

dans lequel la MPSOV comporte un élément de vanne de MPSOV (178) avec une première position dans la MPSOV qui sépare les première et seconde conduites de MPSOV pour ne pas qu'elles soient en communication fluidique et bloque l'écoulement à travers la conduite de sortie, et une seconde position qui raccorde les première et seconde conduites de MPSOV en communication fluidique et permet l'écoulement par la conduite de sortie.

9. Procédé comprenant :

la commande d'une pompe à cylindrée variable, VDP (102), en actionnant un mécanisme à cylindrée variable de la VDP avec un actionneur électromécanique, EMA (136) ;

la réception d'un retour de capteur provenant d'une vanne de détection d'écoulement, FSV, (148) indiquant un écoulement demandé par un système en aval alimenté par une conduite de sortie de la pompe à cylindrée variable, VDP ; et

la commander d'une vanne de dérivation, BPV, (110) avec une vanne d'arrêt de pression minimale, MPSOV, (166) pour recirculer l'écoulement de la conduite de sortie vers une conduite d'entrée de la VDP dans le cas où le retour de capteur tombe en dessous d'un seuil bas prédéterminé d'écoulement à travers la VDP, dans lequel la MPSOV est raccordée pour arrêter sélectivement l'écoulement dans la conduite de sortie ;

dans lequel la commande de la BPV pour recirculer l'écoulement comporte la régulation de l'écoulement de dérivation à travers la BPV selon

$$BF = PF-DSFD$$

dans lequel BF est un écoulement à travers la BPV, PF est un écoulement à travers la VDP, et DSFD est un écoulement demandé par le système en aval alimenté par la conduite de sortie comme indiqué par le retour de capteur.

10. Procédé selon la revendication 9, comprenant en outre la commande de la BPV pour recirculer l'écoulement de la conduite de sortie vers la conduite d'entrée à un taux de recirculation de base constant dans le cas où un écoulement demandé par le système en aval serait égal ou supérieur au seuil bas prédéterminé d'écoulement à travers la VDP ; et éventuellement dans lequel le taux de recirculation de base est un écoulement de recirculation nul.

11. Procédé selon la revendication 9, comprenant en outre la réception de données de pression provenant d'un capteur de pression (146) dans la conduite de sortie, dans lequel la commande de la BPV pour recirculer l'écoulement comporte la commande de la BPV en fonction au moins en partie des données de pression.

12. Procédé selon la revendication 9, comprenant en outre la réception de données provenant d'un capteur indiquant une position d'un élément de vanne de la BPV, dans lequel la commande de la BPV comporte la commande de la BPV en fonction d'une position de l'élément de vanne.

Fig. 1

EP 4 450 810 B1

Fig. 2

EP 4 450 810 B1

**EP 4 450 810 B1**

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2018372006 A1
- US 2014072457 A1
- US 20140072457 A1